# EUROPEAN PATENT SPECIFICATION

(11) **EP 3 471 829 B1**
(45) Date of publication and mention of the grant of the patent: **09.12.2026**
(21) Application number: 17815954.7
(22) Date of filing: 15.06.2017
(51) Int. Cl.: A61K 9/00, A61P 31/18, A61F 2/02, A61K 47/34

(54) **DRUG DELIVERY SYSTEM FOR THE DELIVERY OF ANTIVIRAL AGENTS**
WIRKSTOFFFREISETZUNGSSYSTEM ZUR FREISETZUNG VON VIRUZIDEN
SYSTÈME D'ADMINISTRATION DE MÉDICAMENTS POUR L'ADMINISTRATION D'AGENTS ANTIVIRAUX

(30) Priority: 20.06.2016 US 201662352208 P
(43) Date of publication of application: 24.04.2019
(73) Proprietor: Merck Sharp & Dohme LLC, Rahway, New Jersey 07065 (US)
(72) Inventor: BARRETT, Stephanie Elizabeth, West Point, Pennsylvania 19486 (US); GINDY, Marian E., West Point, Pennsylvania 19486 (US); LI, Li, West Point, Pennsylvania 19486 (US); FORSTER, Seth P., West Point, Pennsylvania 19486 (US); TELLER, Ryan S., West Point, Pennsylvania 19486 (US)
(74) Representative: Merck Sharp & Dohme LLC
(86) International application number: PCT/US2017/037583
(87) International publication number: WO 2017/222903

(56) References cited:
- WO-A1-99/40899
- US-A- 4 148 871
- US-A- 5 227 157
- US-A1- 2005 215 512
- US-A1- 2016 159 752
- US-B2- 8 974 808

## Description

### BACKGROUND OF THE INVENTION

The development of highly active antiretroviral therapy (HAART) in the mid 1990's transformed the clinical care of human immunodeficiency virus (HIV) type infection. HAART regimens have proven to be highly effective treatments, significantly decreasing HIV viral load in HIV-infected patients, thereby slowing the evolution of the illness and reducing HIV-related morbidity and mortality. Yet, the treatment success of HAART is directly related to adherence to the regimen by the patient. Unless appropriate levels of the antiretroviral drug combinations are maintained in the blood, viral mutations will develop, leading to therapy resistance and cross-resistances to molecules of the same therapeutic class, thus placing the long-term efficacy of treatments at risk. Various clinical studies have shown a decline in treatment effectiveness with relatively small lapses in adherence. A study by Musiime found that 81% of patients with more than 95% adherence demonstrated viral suppression, while only 50% of patients who were 80-90% adherent were successful. See, Musiime, S., et al., Adherence to Highly Active Antiretroviral Treatment in HIV-Infected Rwandan Women. PLOS one 2011, 6, (11), 1-6. Remarkably, only 6% of patients that were less than 70% adherent showed improvements in viral markers. Thus, low adherence is a leading cause of therapeutic failure in treatment of HIV-1 infection.

Nonetheless, adherence rates to the HAART regimens continue to be far from optimal. Various characteristics of HAART make adherence particularly difficult. Therapeutic regimens are complex, requiring multiple drugs to be taken daily, often at different times of the day, and many with strict requirements on food intake. Many HAART medications also have unpleasant side effects, including nausea, diarrhea, headache, and peripheral neuropathy. Social and psychological factors can also negatively impact adherence. Patients report that forgetfulness, lifestyle factors, including fear of being identified as HIV-positive, and therapy fatigue over life-long duration of treatment all contribute to adherence lapses.

New HIV treatment interventions aim to improve adherence by reducing the complexity of treatments, the frequency of the dosages, and/or the side effects of the medications. Long-acting injectable (LAI) drug formulations that permit less frequent dosing, on the order of a month or longer, are an increasingly attractive option to address adherence challenges. However, the majority of approved and investigational antiretroviral agents are not well suited for reformulation as long-acting injectable products. In large part, this is due to suboptimal physicochemical properties limiting their formulation as conventional drug suspensions, as well as insufficient antiviral potency resulting in high monthly dosing requirements. Even for cabotegravir or rilpivirine, two drugs being studied as long-acting injectible formulations, large injection volumes and multiple injections are required to achieve pharmacokinetic profiles supportive of monthly dosing. See, e.g., Spreen, W. R., et al., Long-acting injectable antiretrovirals for HIV treatment and prevention. Current Opinion in Hiv and Aids 2013, 8, (6), 565-571; Rajoli, R. K. R., et al., Physiologically Based Pharmacokinetic Modelling to Inform Development of Intramuscular Long-Acting Nanoformulations for HIV. Clinical Pharmacokinetics 2015, 54, (6), 639-650; Baert, L., et al., Development of a long-acting injectable formulation with nanoparticles of rilpivirine (TMC278) for HIV treatment. European Journal of Pharmaceutics and Biopharmaceutics 2009, 72, (3), 502-508; Van 't Klooster, G., et al., Pharmacokinetics and Disposition of Rilpivirine (TMC278) Nanosuspension as a Long-Acting Injectable Antiretroviral Formulation. Antimicrobial Agents and Chemotherapy 2010, 54, (5), 2042-2050. Thus, novel formulation approaches capable of delivering extended-duration pharmacokinetic characteristics for molecules of diverse physicochemical properties at practical injection volumes and with a limited number of injections are highly desirable.

### SUMMARY OF THE INVENTION

This invention relates to novel implant drug delivery systems for long-acting delivery of antiviral drugs as defined in the appended claims. These compositions are useful for the treatment or prevention of human immunodeficiency virus (HIV) infection.

### DETAILED DESCRIPTION OF THE INVENTION

This invention relates to novel implant drug delivery systems for long-acting delivery of antiviral drugs as defined in the appended claims. The novel implant drug delivery systems comprise a polymer and an antiviral agent. These implant drug delivery systems are useful for the treatment or prevention of human immunodeficiency virus (HIV) infection. The invention further relates to the novel implant drug delivery system described herein for use in treating and preventing HIV infection.

The novel implant delivery systems of the invention comprise a biocompatible bioerodible polymer to generate monolithic matrices with dispersed or dissolved drug. The chemical properties of the polymer matrices are tuned to achieve a range of drug release characteristics, offering the opportunity to extend duration of dosing. In an embodiment of the invention, the novel implant delivery systems are compatible with molecules having a broad spectrum of physicochemical properties, including those of high aqueous solubility or amorphous phases which are unsuitable to formulation as solid drug suspensions.

Specifically, this invention relates to novel implant drug delivery systems comprising a biocompatible bioerodible polymer and 4'-ethynyl-2-fluoro-2'-deoxyadenosine, wherein the biocompatible bioerodible polymer is selected from the group consisting of poly(DL-lactide), poly(caprolactone) and combinations thereof, and wherein 4'-ethynyl-2-fluoro-2'-deoxyadenosine is present in the biocompatible bioerodible polymer at 20%-65% by weight of drug loading. These implant delivery systems are desired and useful for prophylaxis and/or treatment of HIV infection from both compliance and convenience standpoints.

As used herein, the term "biocompatible bioerodible polymer" refers to polymeric materials that include hydrolytically labile linkages which undergo cleavage at physiological conditions. The broken down -products are non-toxic and either excreted in the urine or incorporated into the Krebs cycle and used for energy. The polymer is generally hydrophobic so that it retains its integrity for a suitable period of time when placed in an aqueous environment, such as the body of a mammal, and is stable enough to be stored for an extended period before use. Bioerodible polymers remain intact *in vivo* for extended periods of time, typically weeks, months or years. Drug molecules encapsulated in the polymer are released over time via diffusion through channels and pores in a sustained manner. The release rate can be altered by modifying the identity of the polymer (monomeric units, molecular weight, end group, etc.) thereby modifying the degradation kinetics, percent drug loading, porosity of the polymer, structure of the implantable device, or hydrophobicity of the polymer, or by adding a hydrophobic coating to the exterior of the implantable device.

Accordingly, any polymer that can be readily cleared or eliminated by the body can be used to manufacture the implant drug delivery systems that comprise a biocompatible bioerodible polymer. The term "polymer" can also include copolymers. Biocompatible bioerodible polymers of the instant invention includepoly(DL-lactide) ("PLA"), poly(caprolactone) ("PCL") and combinations thereof. In a class of the invention, the biocompatible bioerodible polymer is selected from the group consisting of poly(DL-lactide) and poly(caprolactone).

In a subclass of the invention, the biocompatible bioerodible polymer is poly(DL-lactide). In another subclass of the invention, the biocompatible bioerodible polymer is poly(caprolactone).

As used herein, the term "diffusional barrier" refers to a coating that is permeable to the drug and is placed over at least a portion of the device to further regulate the rate of release. For example, a coating of biocompatible bioerodible polymeric material, e.g., poly(DL-lactide), or a coating of a biocompatible bioerodible polymeric material with a lower drug loading than the remainder of the implant delivery system, may be used. The diffusional barrier may be formed, for example, by coextrusion with the device.

Suitable diffusional barriers of the instant invention include, but are not limited to, poly(DL-lactide) ("PLA"), poly(caprolactone) ("PCL"), poly(lactide-co-glycolide), poly(lactide), poly(glycolide), poly(ortho esters), poly(dioxanone), poly(alkylcyanoacrylates) and combinations thereof. In a class of the invention, the diffusional barrier is selected from the group consisting of poly(DL-lactide) and poly(caprolactone).

In an embodiment of the invention, the diffusion barrier contains an antiviral drug. In a class of the embodiment, the diffusion barrier comprises 4'-ethynyl-2-fluoro-2'-deoxyadenosine.

As used herein, the term "dispersed or dissolved in the biocompatible bioerodible polymer" refers to the drug and polymer being mixed and then hot-melt extruded.

As used herein, the term "continually released" refers to the drug being released from the biocompatible bioerodible polymer at continuous rates for extended periods of time. The implant drug delivery systems of the instant invention generally exhibit 1^{st} order release kinetics for the drug *in vivo,* sometimes with an initial burst. Polymer degradation modifies the dissolution and diffusion of the drug from the polymer matrix. This typically results in an increased drug elution rate that may deviate from 1^{st} order kinetics, and is a function of the polymer degradation rate.

Optionally, the novel implant delivery systems of the instant invention can further comprise a radiopaque component. The radiopaque component will cause the implant to be X-ray visible. The radiopaque component can be any such element known in the art, such as barium sulfate, titanium dioxide, bismuth oxide, tantalum, tungsten or platinum. In a specific embodiment, the radiopaque component is barium sulfate.

In one embodiment, the radiopaque material is about 1% to 30% by weight. In another embodiment, the radiopaque material is about 1% to 20% by weight. In another embodiment, the radiopaque material is about 4% to 25% by weight. In further embodiment, the radiopaque material is about 6% to 20% by weight. In another embodiment, the radiopaque material is about 4% to 15% by weight. In another embodiment, the radiopaque material is about 8% to 15% by weight.

The radiopaque material does not affect the release of 4'-ethynyl-2-fluoro-2'-deoxyadenosine from the implant.

The novel implant delivery systems of the invention comprise 4'-ethynyl-2-fluoro-2'-deoxyadenosine as an antiviral agent. In an embodiment of the invention, the antiviral agent is administered as a monotherapy. In another embodiment of the invention, two or more antiviral agents are administered in combination.

An "anti-HIV agent" is any agent which is directly or indirectly effective in the inhibition of HIV reverse transcriptase or another enzyme required for HIV replication or infection, or prophylaxis of HIV infection, and/or the treatment, prophylaxis or delay in the onset or progression of AIDS. It is understood that an anti-HIV agent is effective in treating, preventing, or delaying the onset or progression of HIV infection or AIDS and/or diseases or conditions arising therefrom or associated therewith. Suitable anti-viral agents for use in implant drug delivery systems described herein include, for example, those listed in Table A as follows:

**Table A: Antiviral Agents for Preventing HIV infection or AIDS**

| Name | Type |
|---|---|
| abacavir, ABC, Ziagen^{®} | nRTI |
| abacavir +lamivudine, Epzicom^{®} | nRTI |
| abacavir + lamivudine + zidovudine, Trizivir^{®} | nRTI |
| amprenavir, Agenerase^{®} | PI |
| atazanavir, Reyataz^{®} | PI |
| AZT, zidovudine, azidothymidine, Retrovir^{®} | nRTI |
| Capravirine | nnRTI |
| darunavir, Prezista^{®} | PI |
| ddC, zalcitabine, dideoxycytidine, Hivid^{®} | nRTI |
| ddI, didanosine, dideoxyinosine, Videx^{®} | nRTI |
| ddI (enteric coated), Videx EC^{®} | nRTI |
| delavirdine, DLV, Rescriptor^{®} | nnRTI |
| doravirine | nnRTI |
| efavirenz, EFV, Sustiva^{®}, Stocrin^{®} | nnRTI |
| efavirenz + emtricitabine + tenofovir DF, Atripla^{®} | nnRTI + nRTI |
| EFdA (4'-ethynyl-2-fluoro-2'-deoxyadenosine) | nRTI |
| emtricitabine, FTC, Emtriva^{®} | nRTI |
| emtricitabine + tenofovir DF, Truvada^{®} | nRTI |
| emvirine, Coactinon^{®} | nnRTI |
| enfuvirtide, Fuzeon^{®} | FI |
| enteric coated didanosine, Videx EC^{®} | nRTI |
| etravirine, TMC-125 | nnRTI |
| fosamprenavir calcium, Lexiva^{®} | PI |
| indinavir, Crixivan^{®} | PI |
| lamivudine, 3TC, Epivir^{®} | nRTI |
| lamivudine + zidovudine, Combivir^{®} | nRTI |
| Lopinavir | PI |
| lopinavir + ritonavir, Kaletra^{®} | PI |
| maraviroc, Selzentry^{®} | EI |
| nelfinavir, Viracept^{®} | PI |
| nevirapine, NVP, Viramune^{®} | nnRTI |
| PPL-100 (also known as PL-462) (Ambrilia) | PI |
| raltegravir, Isentress^{™} | InI |
| (S)-2-(3-chloro-4-fluorobenzyl)-8-ethyl-10-hydroxy-N,6-dimethyl-1,9-dioxo-1,2,6,7,8,9-hexahydropyrazino[1',2':1,5]pyrrolo[2,3-d]pyridazine-4-carboxamide (MK-2048) | InI |
| ritonavir, Norvir^{®} | PI |
| saquinavir, Invirase^{®}, Fortovase^{®} | PI |
| stavudine, d4T,didehydrodeoxythymidine, Zerit^{®} | nRTI |
| tenofovir DF (DF = disoproxil fumarate), TDF, Viread^{®} | nRTI |
| Tenofovir, hexadecyloxypropyl (CMX-157) | nRTI |
| tipranavir, Aptivus^{®} | PI |
| Vicriviroc | EI |

| | |
|---|---|
| EI = entry inhibitor; FI = fusion inhibitor; InI = integrase inhibitor; PI = protease inhibitor; nRTI = nucleoside reverse transcriptase inhibitor; nnRTI = non-nucleoside reverse transcriptase inhibitor. | |

Some of the drugs listed in the table can be used in a salt form; e.g., abacavir sulfate, delavirdine mesylate, indinavir sulfate, atazanavir sulfate, nelfinavir mesylate, saquinavir mesylate.

In certain embodiments the antiviral agents in the implant drug delivery systems described herein are employed in their conventional dosage ranges and regimens as reported in the art, including, for example, the dosages described in editions of the Physicians' Desk Reference, such as the 70th edition (2016) and earlier editions. In other embodiments, the antiviral agents in the implant drug delivery systems described herein are employed in lower than their conventional dosage ranges.

4'-ethynyl-2-fluoro-2'-deoxyadenosine is also known as EFdA, and has the following chemical structure:

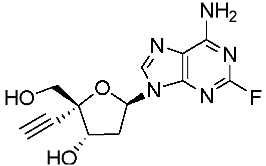

Production of and the ability of 4'-ethynyl-2-fluoro-2'-deoxyadenosine to inhibit HIV reverse transcriptase are described in PCT International Application WO2005090349, published on September 29, 2005, and US Patent Application Publication No. 2005/0215512, published on September 29, 2005, both to Yamasa Corporation.

In an embodiment of the implant drug delivery system described herein, the antiviral agent is present in the biocompatible bioerodible polymer at 20%-65% by weight of drug loading. In other embodiments, the antiviral agent is present in the biocompatible bioerodible polymer at about 20%-60% by weight, at about 30%-65% by weight, at about 40%-60% by weight or at about 40%-45% by weight of drug loading. In an example of the embodiment of the implant drug delivery system described herein, 4'-ethynyl-2-fluoro-2'-deoxyadenosine is present in the biocompatible bioerodible polymer at 40% by weight of drug loading. In another example of the embodiment of the implant drug delivery system described herein, 4'-ethynyl-2-fluoro-2'-deoxyadenosine is present in the biocompatible bioerodible polymer at 45% by weight of drug loading. In another example of the embodiment of the implant drug delivery system described herein, 4'-ethynyl-2-fluoro-2'-deoxyadenosine is present in the biocompatible bioerodible polymer at 50% by weight of drug loading. In another example of the embodiment of the implant drug delivery system described herein, 4'-ethynyl-2-fluoro-2'-deoxyadenosine is present in the biocompatible bioerodible polymer at 60% by weight of drug loading.

The implant drug delivery systems of the instant invention may be produced using an extrusion process, wherein ground biocompatible, bioerodible polymer is blended with the antiviral agent, melted and extruded into rod-shaped structures. Rods are cut into individual implantable devices of the desired length, packaged and sterilized prior to use. Other methods for encapsulating therapeutic compounds in implantable polymeric, bioerodible matrices are known to those of skill in the art. Such methods include solvent casting (see US Patent Nos. 4,883,666, 5,114,719 and 5,601835). One of skill in the art would be able to readily determine an appropriate method of preparing such an implant drug delivery system, depending on the shape, size, drug loading, and release kinetics desired for a particular type of patient or clinical application.

The size and shape of the implant drug delivery systems may be modified to achieve a desired overall dosage. The implant drug delivery systems of the instant invention are often about 0.5 cm to about 10 cm in length. In an embodiment of the invention, the implant drug delivery systems are about 1.5 cm to about 5 cm in length. In a class of the embodiment, the implant drug delivery systems are about 2 cm to about 5 cm in length. In a subclass of the embodiment, the implant drug delivery systems are about 2 cm to about 4 cm in length. The implant drug delivery systems of the instant invention are often about 0.5 mm to about 7 mm in diameter. In an embodiment of the invention, the implant drug delivery systems are about 1.5 mm to about 5 mm in diameter. In a class of the embodiment, the implant drug delivery systems are about 2 mm to about 5 mm in diameter. In a subclass of the embodiment, the implant drug delivery systems are about 2 mm to about 4 mm in diameter.

The implant drug delivery systems described herein are capable of releasing 4'-ethynyl-2-fluoro-2'-deoxyadenosine over a period of 21 days, 28 days, 31 days, 4 weeks, 6 weeks, 8 weeks, 12 weeks, one month, two months, three months, four months, five months, six months, seven months, eight months, nine months, ten months, eleven months, twelve months, eighteen months, twenty-four months or thirty-six months at an average rate of between 0.01-5 mg per day. In an embodiment of the invention, the 4'-ethynyl-2-fluoro-2'-deoxyadenosine is released at therapeutic concentrations for a duration from between three months and thirty-six months. In a class of the embodiment, the 4'-ethynyl-2-fluoro-2'-deoxyadenosine is released at therapeutic concentrations for a duration from between six months and twelve months.

One or more implants can be used to achieve the desired therapeutic dose. In an embodiment of the invention, one or more implants can be used to achieve the therapeutic dose for durations of up to 1 year. In another embodiment of the invention, one or more implants can be used to achieve the therapeutic dose for durations of up to 2 years.

The implant drug delivery systems described herein are capable of releasing 4'-ethynyl-2-fluoro-2'-deoxyadenosine resulting in a plasma concentration of between 0.02-300 ng/mL per day. In an embodiment of the invention, the implant drug delivery systems described herein are capable of releasing 4'-ethynyl-2-fluoro-2'-deoxyadenosine resulting in a plasma concentration of between 0.02-30.0 ng/mL per day. In a class of the embodiment, the implant drug delivery systems described herein are capable of releasing 4'-ethynyl-2-fluoro-2'-deoxyadenosine resulting in a plasma concentration of between 0.02-15 ng/mL per day. In a class of the embodiment, the implant drug delivery systems described herein are capable of releasing 4'-ethynyl-2-fluoro-2'-deoxyadenosine resulting in a plasma concentration of between 0.02-8 ng/mL per day. In a subclass of the embodiment, the implant drug delivery systems described herein are capable of releasing 4'-ethynyl-2-fluoro-2'-deoxyadenosine resulting in a plasma concentration of between 0.1-1.0 ng/mL per day.

The following examples are given for the purpose of illustrating the present invention and shall not be construed as being limitations on the scope of the invention.

### EXAMPLE 1

### PREPARATION AND IN VITRO RELEASE OF IMPLANT DRUG DELIVERY SYSTEMS CONTAINING 40-80 WT% 4'-ETHYNYL-2-FLUORO-2'-DEOXYADENOSINE

Implantable devices were prepared using an extrusion process. The first step involved mixing the dry, micronized powders of the active compound (4'-ethynyl-2-fluoro-2'-deoxyadenosine) and the cryomilled PCL (Evonik RESOMER^{®} Select 100 CL 7.5E) or PLA (Evonik RESOMER^{®} Select 100 DL 8A) using a Turbula T2F mixer. Drug and polymer blends were prepared at 40-80 wt % drug load. The drug and polymer blends were hot-melt extruded using a twin screw extruder through a 3 mm diameter die and pulled to a diameter of approximately 1.9-2.3 mm. The screws contained predominately conveying elements with a single 90° mixing section. The 1^{st} zone where the drug-polymer blends were introduced was water-cooled and maintained at room temperature. The temperature for zones 2-4 was 100°C for PLA and 75°C for PCL. Extruded fibers with diameters between 1.9-2.3 mm were cut to a length of approximately 40 mm.

The *in vitro* release rate of 4'-ethynyl-2-fluoro-2'-deoxyadenosine was determined by incubating the implants segments, approximately 1 cm in length, in a glass vial containing phosphate buffered saline (PBS) at 37°C, and 50 rpm shaking in an Innova 42 incubator. The volume of PBS was sufficient to maintain sink conditions. Sink conditions are defined as the drug concentration maintained at or below 1/3 of the maximum solubility (drug concentration ≤ 0.45 mg/mL in PBS at 37°C). Samples were removed (0.5 mL) at selected time points, and centrifuged at 20,800xg for 8 min. The supernatant was removed (0.4 mL), diluted 4-fold, and vortexed. Samples were assayed by HPLC (Agilent 1100 series). Analysis of a 6 µL volume was performed at 240 nm with a Supelco Ascentis^{®} Express C18 column (100 x 4.6 mm, 2.7µm). The mobile phase was 0.1% H₃PO₄ and 50:50 ACN:MeOH (83:17 v/v) at a flow rate of 1.5 mL/min (40°C).

To determine degradation of 4'-ethynyl-2-fluoro-2'-deoxyadenosine by HPLC, a 6 µL volume was injected onto an Agilent Zorbax SB-Aq column (150 x 4.6 mm, 3.5µm). The mobile phase was 0.1% H₃PO₄ and 50:50 ACN:MeOH with a flow rate of 1.0 mL/min (40°C). The mobile phase gradient is shown in the table below.

**Table 1. 4'-ethynyl-2-fluoro-2'-deoxyadenosine chemical stability HPLC method details**

| **Time (min)** | **0.1 % H₃PO₄** |
|---|---|
| 0.0 | 98 |
| 10.0 | 95 |
| 12.0 | 90 |
| 14.0 | 10 |
| 14.1 | 98 |
| 20.0 | 98 |

All samples were calibrated to 0.5 mg/mL standard solutions of 4'-ethynyl-2-fluoro-2'-deoxyadenosine in 50:50 MeOH:H₂O.

The formulation in Table 2 comprising 80 wt% of the active agent is not in the scope of the claims.

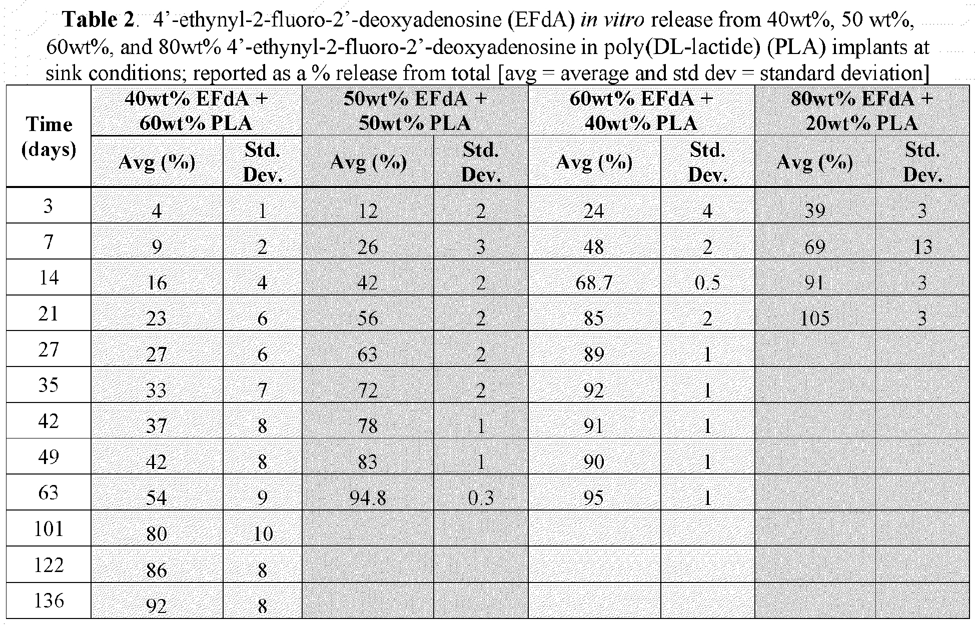

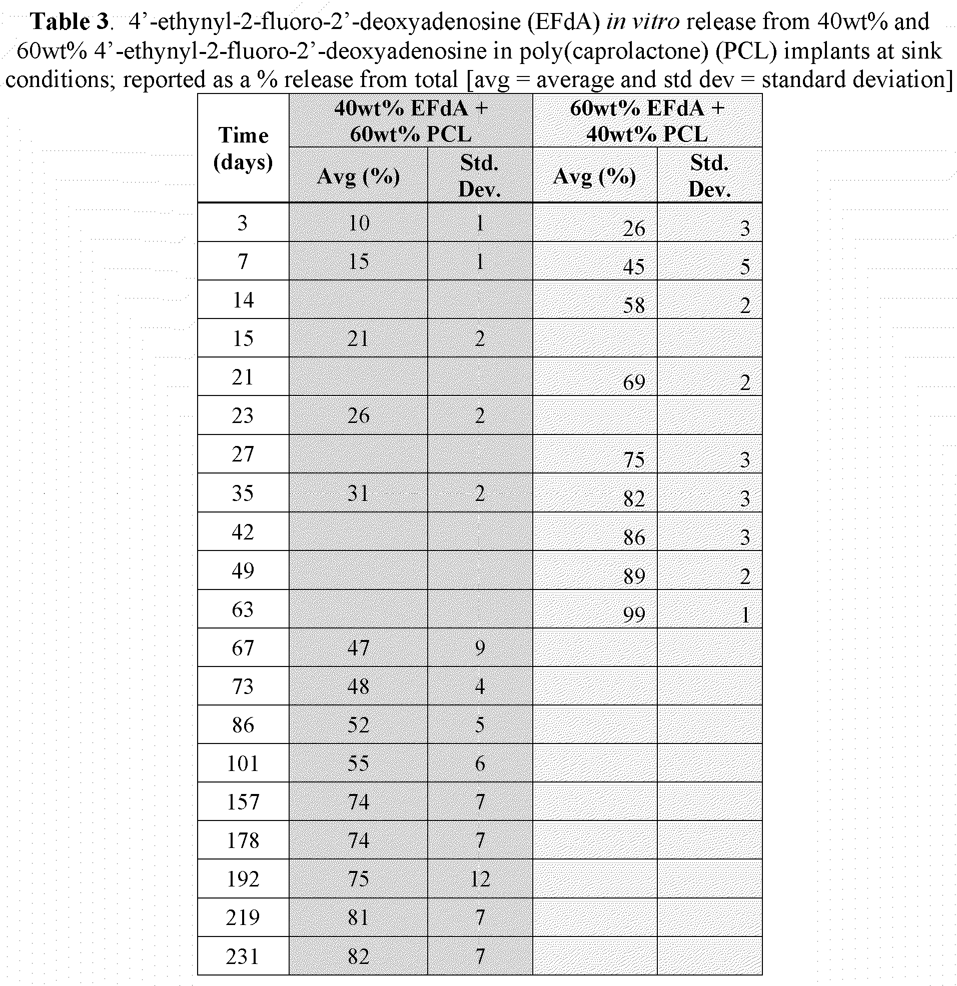

### EXAMPLE 2

### IMPLANT DRUG DELIVERY SYSTEMS WITH DURATIONS OF ACTION OF 1 WEEK TO 3 YEARS

Implantable devices were prepared using an extrusion process. The first step involved mixing the dry, micronized powders of the active compound (4'-ethynyl-2-fluoro-2'-deoxyadenosine) and the cryomilled PCL (Evonik RESOMER^{®} Select 100 CL 7.5E) or PLA (Evonik RESOMER^{®} Select 100 DL 8A) using a Turbula T2F mixer. Drug and polymer blends were prepared at 40-80 wt % drug load. The drug and polymer blends were hot-melt extruded using a twin screw extruder through a 3 mm diameter die and pulled to a diameter of approximately 1.9-2.3 mm. The screws contained predominately conveying elements with a single 90° mixing section. The 1^{st} zone where the drug-polymer blends were introduced was water-cooled and maintained at room temperature. The temperature for zones 2-4 was 100°C for PLA and 75°C for PCL. Extruded fibers with diameters between 1.9-2.3 mm were cut to the appropriate length to achieve the desired amount of drug per implant for *in vivo* studies.

All animal studies were conducted following protocols in accordance with the Institutional Animal Care and Use Committee (IACUC) at NIRC and Merck & Co., Inc., Kenilworth, New Jersey, U.S.A. which adhere to the regulations outlined in the USDA Animal Welfare Act. For each implantation, a Wistar Han rat was anesthetized using isoflurane to effect prior to subcutaneous dose administrations. Using a trocar needle, the solid formulation (~2mm in diameter and of varying lengths based on the body weight of the individual animal to achieve the dose appropriate for each group) was placed in the scapular region. Four animals (2 males and 2 females) were used for each formulation. Animals were monitored until recovered. At indicated time points, samples of blood were obtained from anesthetized animals (using isoflurane) and processed to plasma for determination of 4'-ethynyl-2-fluoro-2'-deoxyadenosine levels.

The formulation in Table 4 comprising 80 wt% of the active agent is not in the scope of the claims.

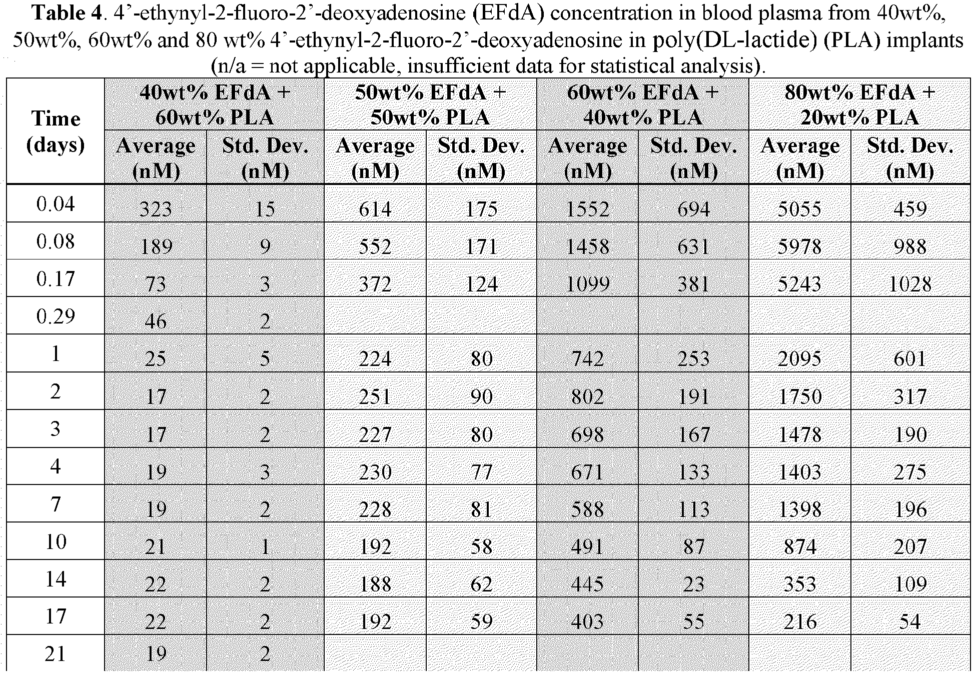

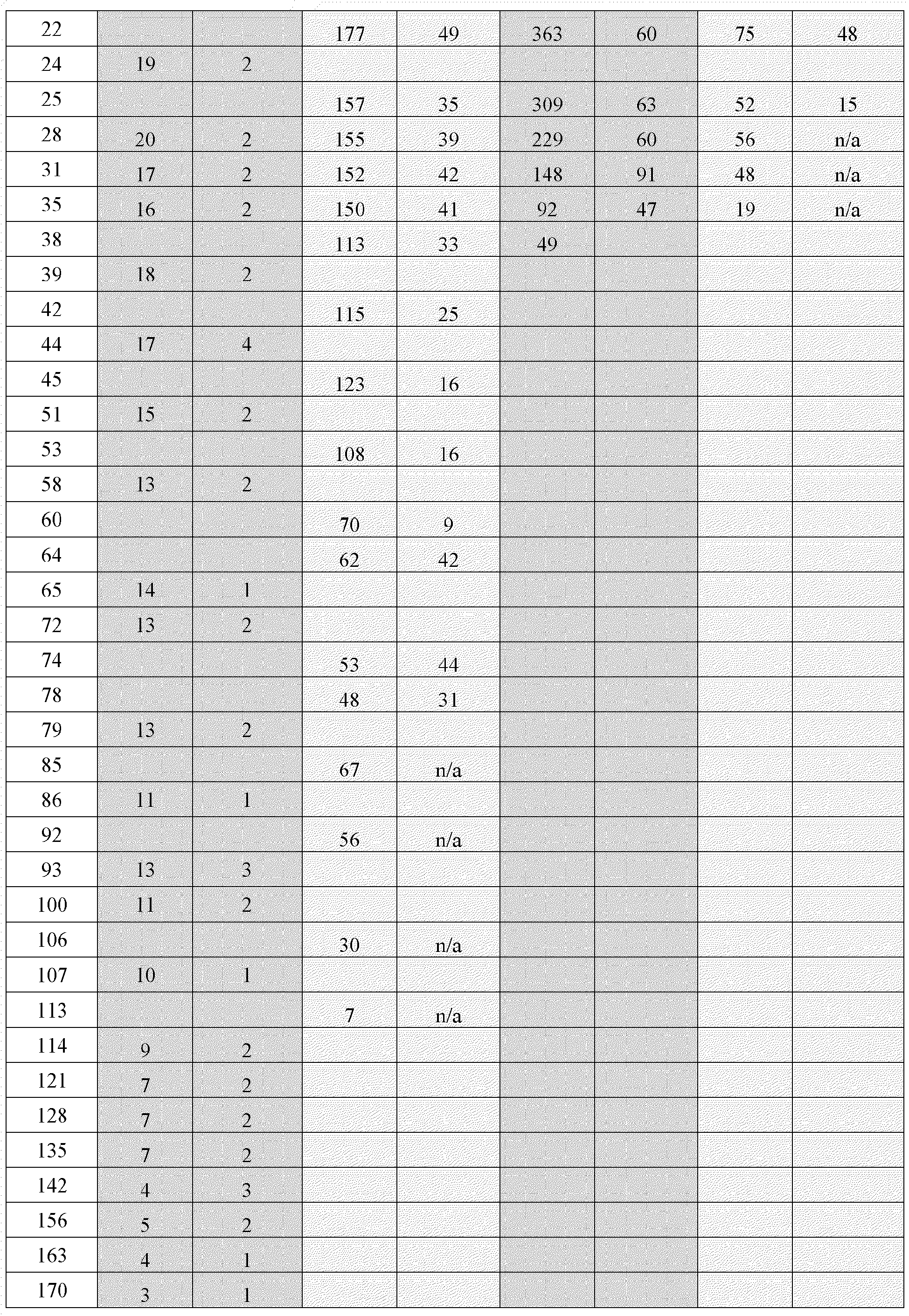

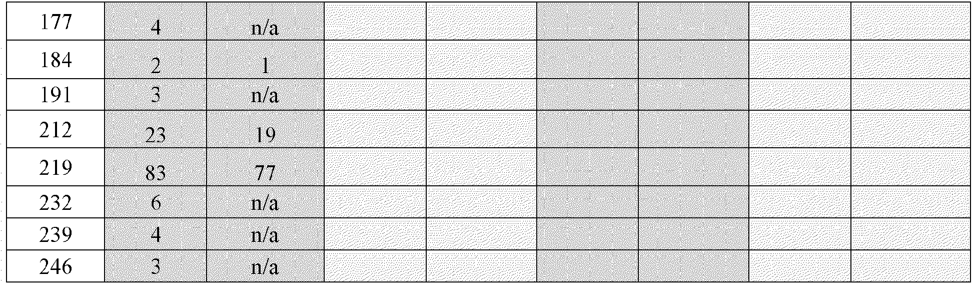

The formulation in Table 5 comprising 80 wt% of the active agent is not in the scope of the claims.

**Table 5. 4'-ethynyl-2-fluoro-2'-deoxyadenosine (EFdA) in vivo release rates and fraction aborbed from 40wt%, 50wt%, 60wt % and 80 wt% 4'-ethynyl-2-fluoro-2'-deoxyadenosine in poly(DL-lactide) (PLA) implants (normalized to a 40 mm long and 2 mm diameter implant)**

| **Sample** | **Release Rate at day 3 (mg/day)** | **Fraction absorbed at day 3 (%)** | **Release Rate at day 30 (mg/day)** | **Fraction absorbed at day 30 (%)** | **Release Rate at day 60 (mg/day)** | **Fraction absorbed at day 60 (%)** | **Release Rate at day 127 (mg/day)** | **Fraction absorbed at day 127 (%)** |
|---|---|---|---|---|---|---|---|---|
| 40wt% EFdA + 60wt% PLA | 0.15 | 1.3 | 0.16 | 8.6 | 0.12 | 15.0 | 0.06 | 25.0 |
| 50wt% EFdA + 50wt% PLA | 0.92 | 3.5 | 0.62 | 26.5 | 0.28 | 42.4 | n/a | 42.4 |
| 60wt% EFdA + 40wt% PLA | 2.74 | 9.4 | 0.69 | 53 | n/a | 53 | n/a | 53 |
| 80wt% EFdA + 20wt% PLA | 6.12 | 21.7 | 0.21 (day 25) | 60.7 | n/a | 60.7 | n/a | 60.7 |

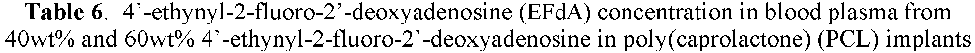

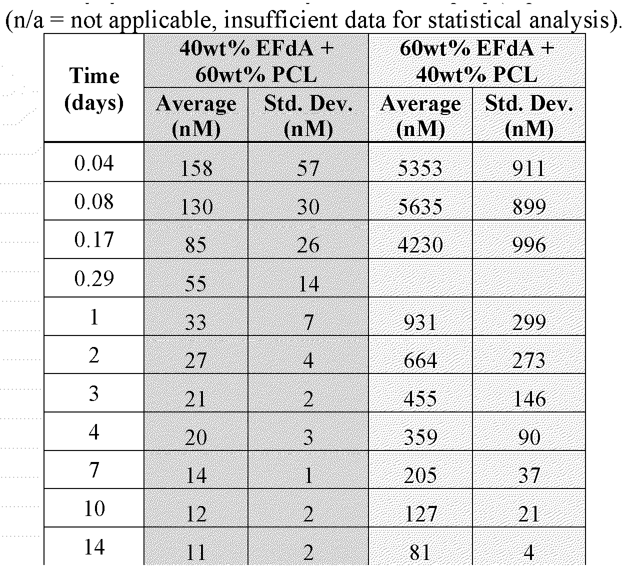

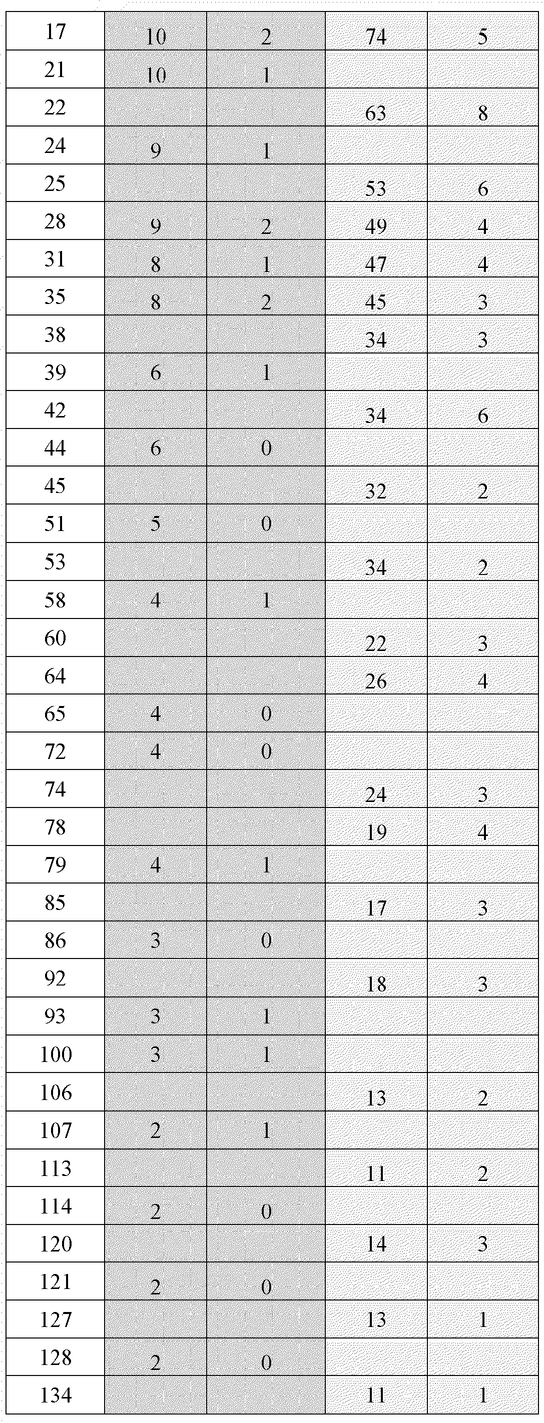

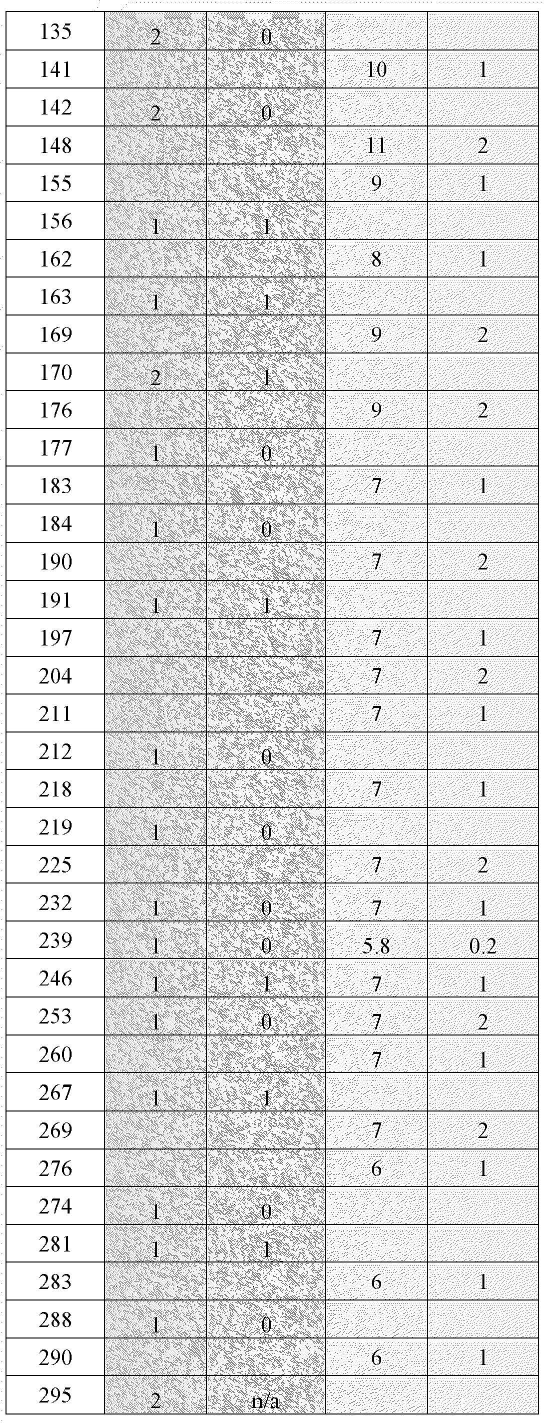

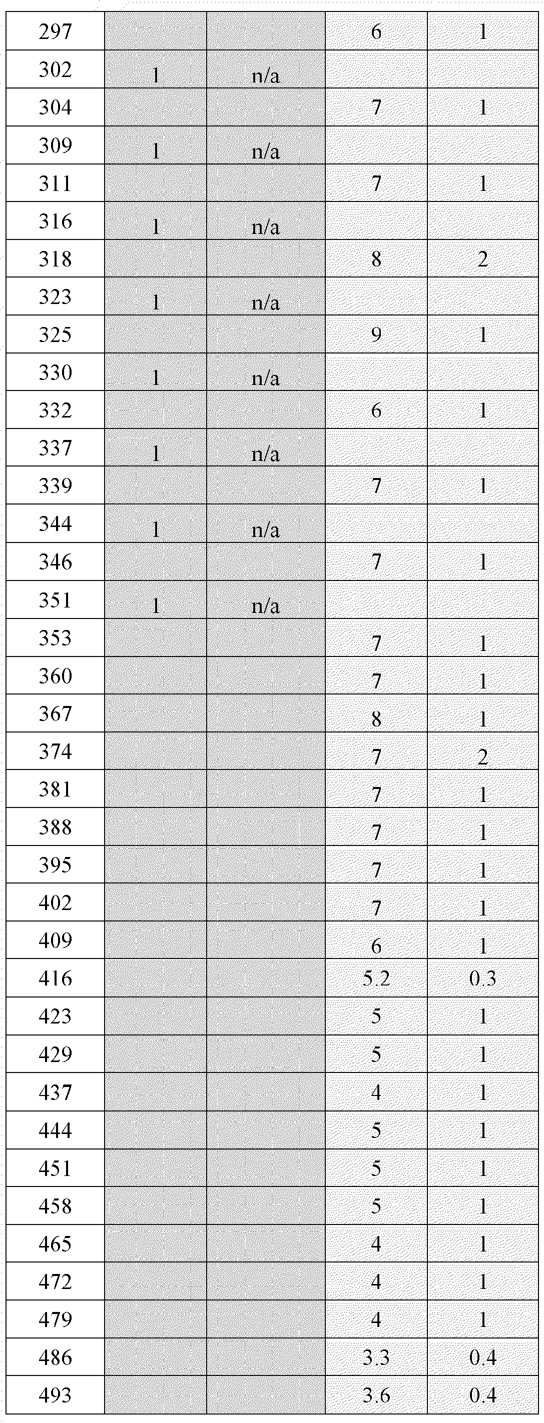

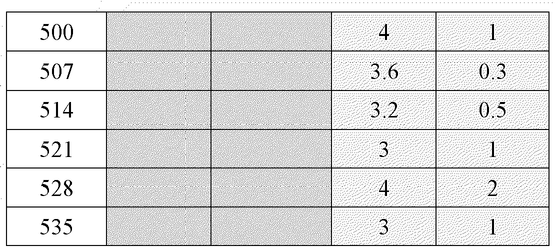

**Table 7. 4'-ethynyl-2-fluoro-2'-deoxyadenosine (EFdA) in vivo release rates and fraction absorbed from 40% and 60% 4'-ethynyl-2-fluoro-2'-deoxyadenosine in poly(caprolactone) (PCL) implants (normalized to a 40 mm long and 2 mm diameter implant)**

| **Sample** | **Release Rate at day 3 (mg/day)** | **Fraction absorbe d at day 3 (%)** | **Release Rate at day 30 (mg/day)** | **Fraction absorbe d at day 30 (%)** | **Release Rate at day 60 (mg/day)** | **Fraction absorbe d at day 60 (%)** | **Release Rate at day 127 (mg/day)** | **Fraction absorbe d at day 127 (%)** |
|---|---|---|---|---|---|---|---|---|
| 40wt% EFdA + 60wt% PCL | 0.58 | 5.3 | 0.23 | 20.1 | 0.12 | 28.9 | 0.05 | 38.6 |
| 60wt% EFdA + 40wt% PCL | 1.69 | 15.6 | 0.19 | 26.9 | 0.09 | 30.8 | 0.05 | 35.1 |

### EXAMPLE 3

### PREPARATION AND IN VIVO STUDIES OF IMPLANT DRUG DELIVERY SYSTEMS CONTAINING 45 WT% 4'-ETHYNYL-2-FLUORO-2'-DEOXYADENOSINE

Implantable devices were prepared using an extrusion process. The first step involved mixing the dry, micronized powders of the active compound (4'-ethynyl-2-fluoro-2'-deoxyadenosine) and the cryomilled PCL (Evonik RESOMER^{®} Select 100 CL 7.5E) or PLA (Evonik RESOMER^{®} Select 100 DL 8A) using a Turbula T2F mixer. Drug and polymer blends were prepared at 40-80 wt % drug load. The drug and polymer blends were hot-melt extruded using a twin screw extruder through a 3 mm diameter die and pulled to a diameter of approximately 1.9-2.3 mm. The screws contained predominately conveying elements with a single 90° mixing section. The 1^{st} zone where the drug-polymer blends were introduced was water-cooled and maintained at room temperature. The temperature for zones 2-4 was 100°C for PLA and 75°C for PCL. Extruded fibers with diameters between 1.9-2.3 mm were cut to the appropriate length to achieve the desired amount of drug per implant for *in vivo* studies.

All animal studies were conducted following protocols in accordance with the Institutional Animal Care and Use Committee (IACUC) at NIRC and Merck, which adhere to the regulations outlined in the USDA Animal Welfare Act. For each implantation, a Rhesus monkey was sedated with Ketamine HCl (100 mg/mL) prior to subcutaneous implant administrations. Using an injector device, the implant was placed subcutaneously in the interscapular region. Either two or three animals were used per group (n=2 for 40wt% EFdA + 60wt% PLA, n=3 for 50wt% EFdA + 50wt% PLA, n=2 for 50wt% EFdA + 50wt% PCL). Animals were monitored until recovered. At indicated time points, samples of blood were obtained and processed to plasma for determination of 4'-ethynyl-2-fluoro-2'-deoxyadenosine levels.

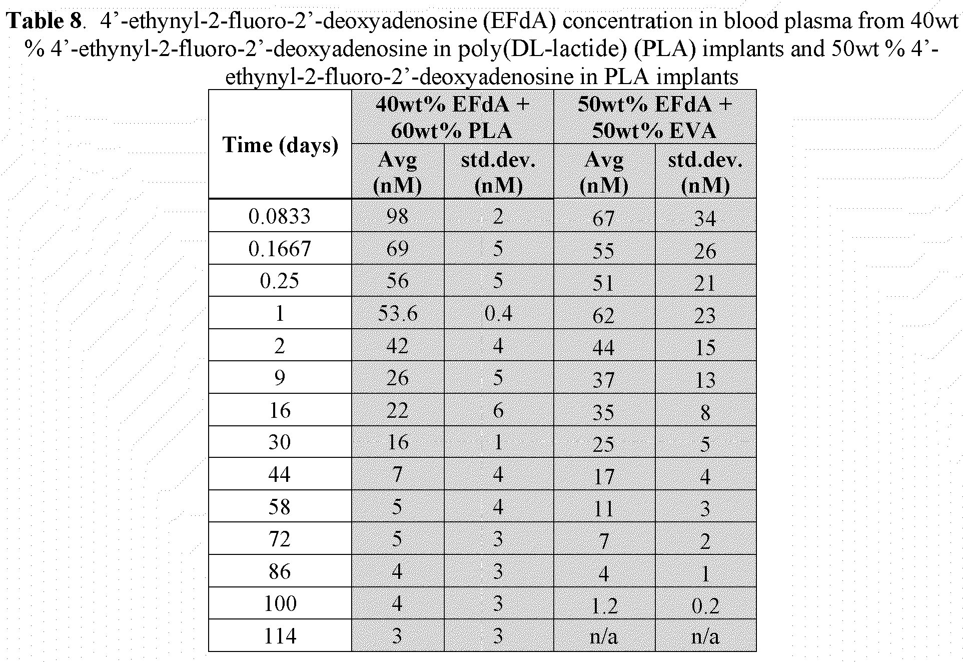

**Table 9. 4'-ethynyl-2-fluoro-2'-deoxyadenosine (EFdA) in vivo release rates from 40wt% and 50wt% 4'-ethynyl-2-fluoro-2'-deoxyadenosine in poly(DL-lactide) (PLA) implants**

| **Sample** | **Release rate at day 30 (mg/day)** | **Release rate at day 60 (mg/day)** | **Release rate at day 114 (mg/day)** |
|---|---|---|---|
| 40wt% EFdA + 60wt%PLA | 0.40-0.74 | 0.14-0.26 | 0.07-0.18 |
| 50wt% EFdA + 50wt%PLA | 0.69-1.29 | 0.30-0.55 | n/a |

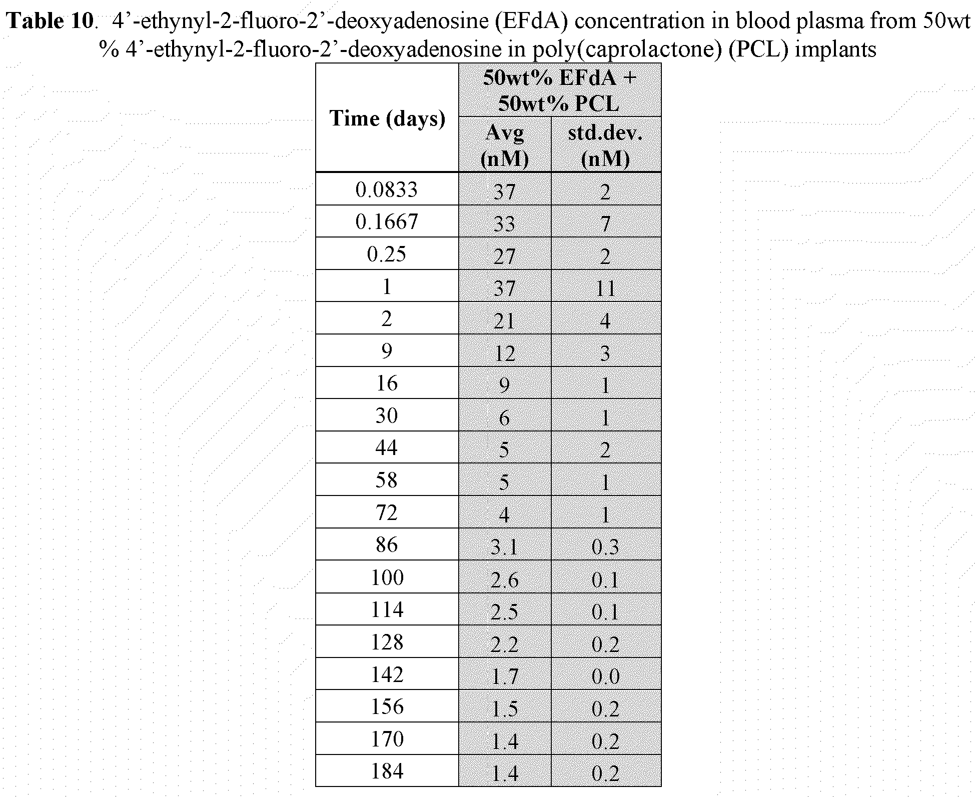

**Table 11. 4'-ethynyl-2-fluoro-2'-deoxyadenosine (EFdA) in vivo release rates from 50wt% 4'-ethynyl-2-fluoro-2'-deoxyadenosine in poly(caprolactone) (PCL) implants**

| **Sample** | **Release rate at day 30 (mg/day)** | **Release rate at day 60 (mg/day)** | **Release rate at day 114 (mg/day)** |
|---|---|---|---|
| 50wt% EFdA + 50wt%PCL | 0.22-0.47 | 0.18-0.38 | 0.09-0.19 |

## Claims

1. An implant drug delivery system comprising a biocompatible bioerodible polymer and 4'-ethynyl-2-fluoro-2'-deoxyadenosine, wherein the biocompatible bioerodible polymer is selected from the group consisting of poly(DL-lactide), poly(caprolactone) and combinations thereof, and wherein 4'-ethynyl-2-fluoro-2'-deoxyadenosine is present in the biocompatible bioerodible polymer at 20%-65% by weight of drug loading.

2. The implant drug delivery system of Claim 1, wherein the biocompatible bioerodible polymer is poly(DL-lactide).

3. The implant drug delivery system of Claim 1, wherein the biocompatible bioerodible polymer is poly(caprolactone).

4. The implant drug delivery system of Claim 1, further comprising a diffusional barrier.

5. The implant drug delivery system of Claim 4, wherein the diffusional barrier is selected from the group consisting of poly(lactide-co-glycolide), poly(lactide), poly(glycolide), poly(caprolactone), poly(ortho esters), poly(dioxanone), poly(alkylcyanoacrylates) and combinations thereof.

6. The implant drug delivery system of Claim 5, wherein the diffusional barrier comprises 4'-ethynyl-2-fluoro-2'-deoxyadenosine.

7. The implant drug delivery system of Claim 1, wherein the 4'-ethynyl-2-fluoro-2'-deoxyadenosine is dispersed or dissolved in the biocompatible bioerodible polymer.

8. The implant drug delivery system of Claim 1, wherein 4'-ethynyl-2-fluoro-2'-deoxyadenosine is present in the biocompatible bioerodible polymer at 20%-60% by weight of drug loading.

9. The implant drug delivery system of Claim 1, wherein 4'-ethynyl-2-fluoro-2'-deoxyadenosine is present in the biocompatible bioerodible polymer at 30%-65% by weight of drug loading.

10. The implant drug delivery system of Claim 8, wherein 4'-ethynyl-2-fluoro-2'-deoxyadenosine is present in the biocompatible bioerodible polymer at 40%-45% by weight of drug loading.

11. The implant drug delivery system of Claim 1, comprising between 1% and 20% by weight of a radiopaque material.

12. The implant drug delivery system according to Claim 1, for use in treating or preventing HIV infection.

## Patentansprüche

1. Ein Implantat-Arzneimittelabgabesystem, umfassend ein biokompatibles, biologisch abbaubares Polymer und 4'-Ethinyl-2-fluor-2'-desoxyadenosin, wobei das biokompatible, biologisch abbaubare Polymer ausgewählt ist aus der Gruppe bestehend aus Poly(DL-lactid), Poly(caprolacton) und Kombinationen davon, und wobei 4'-Ethinyl-2-fluor-2'-desoxyadenosin im biokompatiblen, biologisch abbaubaren Polymer mit einem Wirkstoffgehalt von 20 Gew.-% bis 65 Gew.-% enthalten ist.

2. Das Implantat-Arzneimittelabgabesystem nach Anspruch 1, wobei das biokompatible, biologisch abbaubare Polymer Poly(DL-lactid) ist.

3. Das Implantat-Arzneimittelabgabesystem nach Anspruch 1, wobei das biokompatible, biologisch abbaubare Polymer Poly(caprolacton) ist.

4. Das Implantat-Arzneimittelabgabesystem nach Anspruch 1, ferner umfassend eine Diffusionssperre.

5. Das Implantat-Arzneimittelabgabesystem nach Anspruch 4, wobei die Diffusionssperre ausgewählt ist aus der Gruppe bestehend aus Poly(lactid-co-glycolid), Poly(lactid), Poly(glycolid), Poly(caprolacton), Poly(orthoestern), Poly(dioxanon), Poly(alkylcyanoacrylaten) und Kombinationen davon.

6. Das Implantat-Arzneimittelabgabesystem nach Anspruch 5, wobei die Diffusionssperre 4'-Ethinyl-2-fluor-2'-desoxyadenosin umfasst.

7. Das Implantat-Arzneimittelabgabesystem nach Anspruch 1, wobei das 4'-Ethinyl-2-fluor-2'-desoxyadenosin in dem biokompatiblen, biologisch abbaubaren Polymer dispergiert oder gelöst ist.

8. Das Implantat-Arzneimittelabgabesystem nach Anspruch 1, wobei 4'-Ethinyl-2-fluor-2'-desoxyadenosin in dem biokompatiblen, biologisch abbaubaren Polymer mit einem Wirkstoffgehalt von 20 Gew.-% bis 60 Gew.-% enthalten ist.

9. Das Implantat-Arzneimittelabgabesystem nach Anspruch 1, wobei 4'-Ethinyl-2-fluor-2'-desoxyadenosin in dem biokompatiblen, biologisch abbaubaren Polymer mit einem Wirkstoffgehalt von 30 Gew.-% bis 65 Gew.-% enthalten ist.

10. Das Implantat-Arzneimittelabgabesystem nach Anspruch 8, wobei 4'-Ethinyl-2-fluor-2'-desoxyadenosin in dem biokompatiblen, biologisch abbaubaren Polymer mit einem Wirkstoffgehalt von 40 Gew.-% bis 45 Gew.-% enthalten ist.

11. Das Implantat-Arzneimittelabgabesystem nach Anspruch 1, das zwischen 1 Gew.-% und 20 Gew.-% eines Kontrastmittels umfasst.

12. Das Implantat-Arzneimittelabgabesystem nach Anspruch 1 zur Verwendung bei der Behandlung oder Prävention einer HIV-Infektion.

## Revendications

1. Système d'administration de médicament par implant comprenant un polymère bioérodable biocompatible et de la 4'-éthynyl-2-fluoro-2'-désoxyadénosine, dans lequel le polymère bioérodable biocompatible est choisi dans le groupe constitué par le poly(DL-lactide), la poly(caprolactone) et leurs combinaisons, et dans lequel la 4'-éthynyl-2-fluoro-2'-désoxyadénosine est présente dans le polymère bioérodable à raison de 20 % à 65 % en poids de la charge médicamenteuse.

2. Système d'administration de médicament par implant selon la revendication 1, dans lequel le polymère biocompatible bioérodable est le poly(DL-lactide).

3. Système d'administration de médicament par implant selon la revendication 1, dans lequel le polymère biocompatible bioérodable est la poly(caprolactone).

4. Système d'administration de médicament par implant selon la revendication 1, comprenant en outre une barrière diffusionnelle.

5. Système d'administration de médicament par implant selon la revendication 4, dans lequel la barrière diffusionnelle est choisie dans le groupe constitué par le poly(lactide-co-glycolide), le poly(lactide), le poly(glycolide), la poly(caprolactone), les poly(ortho esters), la poly(dioxanone), les poly(cyanoacrylates d'alkyle) et leurs combinaisons.

6. Système d'administration de médicament par implant selon la revendication 5, dans lequel la barrière diffusionnelle comprend de la 4'-éthynyl-2-fluoro-2'-désoxyadénosine.

7. Système d'administration de médicament par implant selon la revendication 1, dans lequel la 4'-éthynyl-2-fluoro-2'-désoxyadénosine est dispersée ou dissoute dans le polymère biocompatible bioérodable.

8. Système d'administration de médicament par implant de la revendication 1 dans lequel la 4'-éthynyl-2-fluoro-2'-désoxyadénosine est présente dans le polymère bioérodable biocompatible à raison de 20 % à 60 % en poids de la charge médicamenteuse.

9. Système d'administration de médicament par implant de la revendication 1 dans lequel la 4'-éthynyl-2-fluoro-2'-désoxyadénosine est présente dans le polymère bioérodable biocompatible à raison de 30 % à 65 % en poids de la charge médicamenteuse.

10. Système d'administration de médicament par implant de la revendication 8 dans lequel la 4'-éthynyl-2-fluoro-2'-désoxyadénosine est présente dans le polymère bioérodable biocompatible à raison de 40 % à 45 % en poids de la charge médicamenteuse.

11. Système d'administration de médicament par implant de la revendication 1 comprenant entre 1 % et 20 % en poids d'un matériau radio-opaque.

12. Système d'administration de médicament par implant de la revendication 1, destiné à être utilisé dans le traitement ou la prévention d'une infection à VIH.
